(19) Europäisches Patentamt
European Patent Office
Office européen des brevets

(11) Publication number: **0 141 560**
**B1**

(12) **EUROPEAN PATENT SPECIFICATION**

(45) Date of publication of patent specification: **25.01.89**

(51) Int. Cl.[4]: **C 07 D 405/14**

(21) Application number: **84307023.6**

(22) Date of filing: **15.10.84**

(54) **Chemical process.**

(30) Priority: **21.10.83 GB 8328240**

(43) Date of publication of application:
**15.05.85 Bulletin 85/20**

(45) Publication of the grant of the patent:
**25.01.89 Bulletin 89/04**

(84) Designated Contracting States:
**AT BE CH DE FR GB IT LI LU NL SE**

(58) References cited:
**EP-A-0 003 677**
**EP-A-0 103 366**

**The file contains technical information submitted after the application was filed and not included in this specification**

(73) Proprietor: **SMITH KLINE & FRENCH LABORATORIES LIMITED**
**Mundells**
**Welwyn Garden City Hertfordshire, AL7 1EY (GB)**

(72) Inventor: **Adger, Brian Michael**
**8 Tonbridge Road**
**Hildenborough Nr Tonbridge Kent TN9 2NG (GB)**
Inventor: **Lewis, Norman John**
**20A Norton Road Southborough**
**Tunbridge Wells Kent TN4 0HE (GB)**

(74) Representative: **Denerley, Paul Millington, Dr. et al**
**Smith Kline & French Laboratories Ltd Patent Department Mundells**
**Welwyn Garden City Hertfordshire AL7 1EY (GB)**

**EP 0 141 560 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the preparation of a Mannich base which has activity as an histamine $H_2$-antagonist, and is an intermediate in the preparation of another $H_2$-antagonist.

European Patent No 0003677 discloses inter alia a process for the preparation of compounds of the formula (I):—

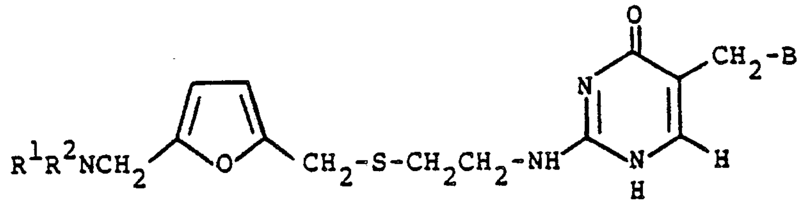

(I)

wherein $R^1$ and $R^2$ are lower alkyl or $R^1$ and $R^2$ together with the nitrogen atom to which they are attached form a pyrrolidino or piperidino group, and B is pyrid-3-yl, 6-methylpyrid-3-yl, 5,6-dimethylpyrid-3-yl, 6-methoxypyrid-3-yl, 2-methoxypyrid-4-yl, 6-hydroxypyrid-3-yl or 2-hydroxypyrid-4-yl; which process comprises the reaction of a compound of the formula (II):—

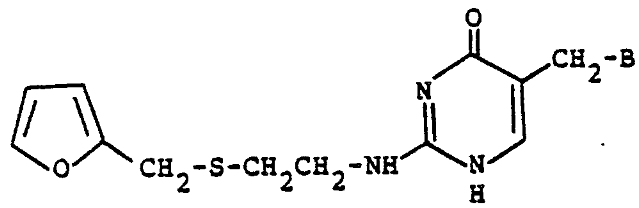

(II)

wherein B is as defined in relation to formula (I), with a Mannich reagent.

We have discovered that when a compound of the formula (II) wherein B is 6-methylpyrid-3-yl is reacted with the Mannich reagent bis(dimethylamino)methane, the purity of the product is sensitive to the conditions under which the reaction is performed. The formation of the desired product, i.e. that of formula (I) wherein $R^1$ and $R^2$ are both methyl and B is 6-methylpyrid-3-yl, under acidic conditions, can be accompanied by 2—3% of a by-product of the formula (III):

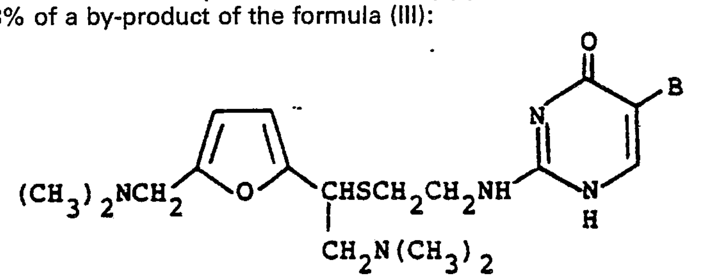

(III)

The desired product and the compound of the formula (III) have chemical structures and properties that make separation by conventional commercially feasible techniques difficult. For example dissolving the product mixture in a standard biphase water-organic solvent system and adjusting the pH of the aqueous layer to the range 6—7 affords good purification, but this leads to a further problem as during this purification procedure, dissolving the desired product in a chlorinated organic solvent, for example dichloromethane, at about neutral pH can form a substantial amount of a quaternary salt of the formula (IV) which is not readily removed:—

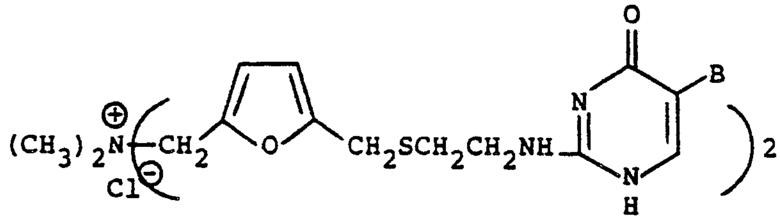

(IV)

In addition at low pH values a major impurity is the thiol of the formula (V) which oxidises to the disulphide.

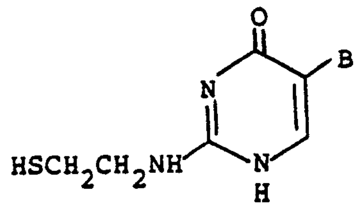

(V)

Furthermore furans are known to decompose at low pH values.

A histamine $H_2$-antagonist of considerable interest is 2-(2-(5-dimethylaminomethylfuran-2-ylmethylthio)ethylamino)-5-(2-hydroxypyrid-4-ylmethyl)pyrimidin-4-one as well as the pharmaceutically acceptable salts thereof. This compound is described in the above mentioned European Patent and can be prepared by reaction with a Mannich reagent and deprotection, if necessary, of 2-(2-furan-2-ylmethylthio)ethylamino)-5-(2-hydroxypyrid-4-ylmethyl)-pyrimidin-4-one wherein the hydroxy group is optionally protected.

The problems identified above with reference to the synthesis of the compound wherein B is 6-methylpyrid-3-ylmethyl group are thought to be present in the syntheses of compounds wherein B is a 2-(optionally protected)-hydroxypyrid-4-ylmethyl group. Thus the synthesis of the desired hydroxypyridyl $H_2$-antagonist comprises the same problems of sensitivity to the reaction conditions, with numerous side-reactions giving undesirably high levels of impurities that cannot be readily removed.

It is an object of the present invention to provide a process that produces the desired protected hydroxypyridyl compound in high yields without any significant impurity, so that it is readily and efficiently handled, for the deprotection reaction, on a substantial scale of synthesis.

Accordingly the present invention provides a process for the preparation of a compound of the formula (VI):—

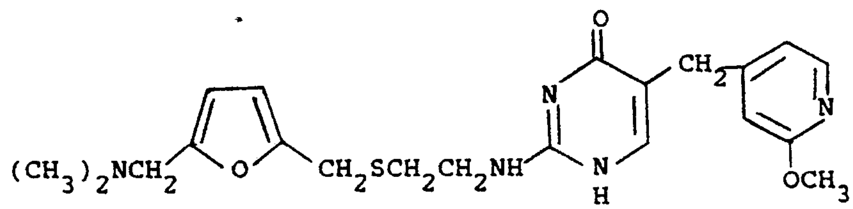

(VI)

which comprises reacting a compound of the formula (VII):—

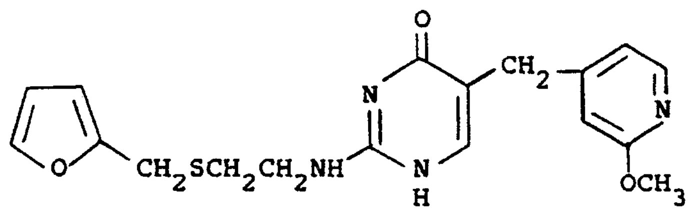

(VII)

with an organic solution of bis(dimethylamino)methane at a pH of 1.3 to 2.5.

Preferably the pH range is 1.7 to 2.3.

When used herein the pH value of the reactant solution is measured by adding at aliquot (0.5 ml) to water (5 ml), shaking, and measuring the pH of the aqueous layer. .

Suitably the process is carried out in a chlorinated organic solvent, for example dichloromethane, dichloroethane, dichloroethane, chloroform or dischlorobenzene. Preferably the process is carried out in dichloromethane. Preferably the process is performed under substantially anhydrous conditions.

It is advantageous to distil the bis(dimethylamino)methane before use.

The required pH of the reaction mixture is conveniently achieved by the controlled addition of gaseous hydrogen chloride to a mixture of the compound of the formula (VI) and bis(dimethylamino)methane in a suitable solvent, both compounds thus being in the form of a salt formed with hydrochloric acid.

Alternatively other methods of acidification may be used, for example hydrogen bromide or methanesulphonic acid may be added. The acidifying agent should be one that gives rise to a counter-ion which would not cause any substantial precipitation of salt with either bis(dimethylamino)methane or the compound of the formula (VII).

The reaction conveniently takes place at a temperature between 0°C and about 80°C, for example most conveniently at ambient or at reflux in dichloromethane. When substantially all of the compound of the formula (VII) has reacted, as determined by conventional assay techniques for example high performance liquid chromatography or thin layer chromatography, the desired product is isolated conveniently by precipitation from an organic solvent for example isopropanol.

The process of this invention has significant advantages over related processes, for example when the corresponding reaction is performed on corresponding compounds of the formula (VII) wherein methoxy is replaced by hydroxy or benzyloxy, then low yields of impure oils or hygroscopic solids were obtained.

The compound of the formula (VI) is active as an $H_2$-antagonist. European Patent 3677 generally

discloses this compound, pharmaceutical compositions containing it and its use as an $H_2$-antagonist. In addition, as stated above, this compound is of major value as an intermediate in the preparation of 2-(2-(5-dimethyl-aminomethylfuran-2-ylmethylthio)ethylamino)-5-(2-hydroxypyrid-4-ylmethyl)pyrimidin-4-one. The conversion of the methoxy group to the hydroxy group can be performed in conventional manner for example by hydrolysis under acidic conditions. The methoxy group may be, at least, partially deprotected during the process of this invention to form the corresponding hydroxy compound. Longer time periods and/or higher temperatures may be required for such deprotection than for the Mannich reaction. Even if deprotection is only partial, such a product mixture is of value as the product mixture can optionally be isolated and further deprotected to form the hydroxy compound.

The following Example illustrates the invention.

Example 1

2-[2-(5-Dimethylaminomethylfuran-2-ylmethylthio)ethylamino]-5-(2-methoxypyrid-4-ylmethyl)pyrimidin-4-one trihydrochloride

i) 2-[2-(Furan-2-ylmethylthio)ethylamino]-5-(2-methoxypyrid-4-ylmethyl)pyrimidin-4-one

2-Nitroamino-5-(2-methoxypyrid-4-ylmethyl)pyrimidin-4-one (36.00 g) and 2-(furan-2-ylmethylthio)-ethylamine (20.29 g) were refluxed in pyridine (200 ml) for 16 hours. The reaction mixture was cooled to ambient temperature and then to ice-bath temperature. The cooled mixture was treated with water (800 ml) and rapidly stirred whereupon crystallisation occurred (may be induced by a seed crystal and/or trituration). The reaction mixture was filtered after 60 minutes and the residue washed with water (100 ml) to afford, on drying 2-[2-(furan-2-ylmethylthio)ethylamino]-5-(2-methoxypyrid-4-ylmethyl)pyrimidin-4-one (43.41 g), m.p. 113—5°C (uncorrected).

ii) 2-[2-(5-Dimethylaminomethylfuran-2-ylmethylthio)-ethylamino]-5-(2-methoxypyrid-4-ylmethyl)pyrimidin-4-one trihydrochloride

2-[2-(Furan-2-ylmethylthio)ethylamino]-5-(2-methoxypyrid-4-ylmethyl)pyrimidin-4-one (8.0 g) was suspended in dichloromethane (80 ml). The resulting mixture was treated with bis(dimethylamino)-methane (6.52 g, 0.064 mol) and the mixture was stirred and cooled in an ice bath whilst hydrogen chloride gas was bubbled into the reaction mixture. The rate of addition of hydrogen chloride was controlled to keep the internal temperature of the reaction mixture below 20°C. Addition of hydrogen chloride gas was continued until a sample of the reaction mixture (0.5 ml) shaken with water (5 ml) showed a pH value of 2 using Merck Acilit pH papers (Acilit is a Trade Mark). The reaction mixture was then allowed to warm to ambient temperature and was stirred until thin layer chromatographic analysis showed no starting material (silica, eluted with ethyl acetate: methanol: 0.880 ammonia 5:1:1); Starting material Rf 0.56, desired product Rf 0,49.

At this time stirring was stopped and the reaction mixture was allowed to settle. The lower layer was removed, and the upper yellow layer was treated with isopropanol (160 ml). The resulting mixture was stirred and cooled in an ice bath for 30 minutes and the precipitated solid was filtered and dried under vacuum at 50°C. This gave the title compound (10.8 g) as a pale yellow solid, m.p. 152—5°C (decomposition).

2-[2-(5-Dimethylaminomethylfuran-2-ylmethylthio)ethylamino]-5-(2-methoxypyrid-4-ylmethyl)-pyrimidin-4-one trihydrochloride is converted to 2-[2-(5-dimethylaminomethylfuran-2-ylmethylthio)ethylamino]-5-(2-hydroxypyrid-4-ylmethyl)pyrimidin-4-one trihydrochloride by treating with hydrogen chloride in ethanol, refluxing for 20 hours, cooling, and evaporating to dryness to give a residue. This residue is crystallised from methanol/ethanol to give the trihydrochloride product.

**Claims**

1. A process for the preparation of a compound of the formula (VI):

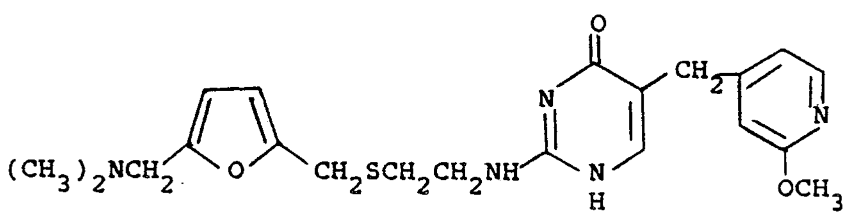

(VI)

which comprises reacting a compound of the formula (VII):—

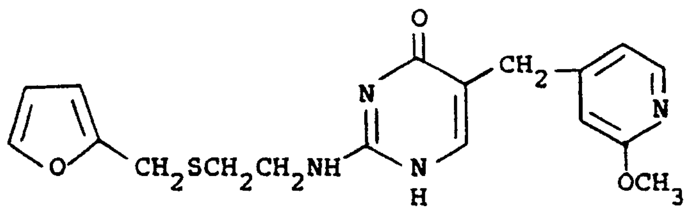

(VII)

with an organic solution of bis(dimethylamino)methane at a pH of 1.3 to 2.5.

2. A process according to claim 1 wherein the pH range is 1.7 to 2.3.

3. A process according to either claim 1 or claim 2 when performed in dichloromethane.

4. A process according to claim 1 wherein the required pH is achieved by the addition of gaseous hydrogen chloride.

5. A process according to claim 1 wherein the compound of the formula (VI) is deprotected to form 2-(2-(5-dimethylaminomethylfuran-2-ylmethylthio)ethylamino)-5-(2-hydroxypyrid-4-ylmethyl)pyrimidin-4-one or a pharmaceutically acceptable salt thereof.

**Patentansprüche**

1. Verfahren zur Herstellung einer Verbindung der Formel VI

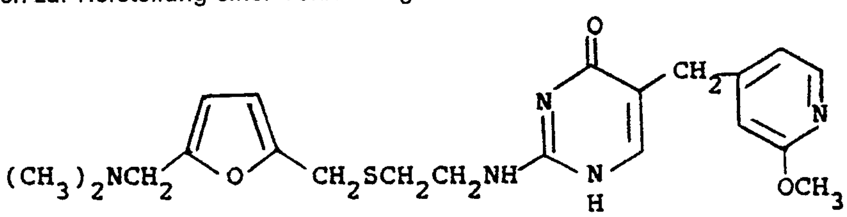

(VI)

bei dem man eine Verbindung der Formel VII

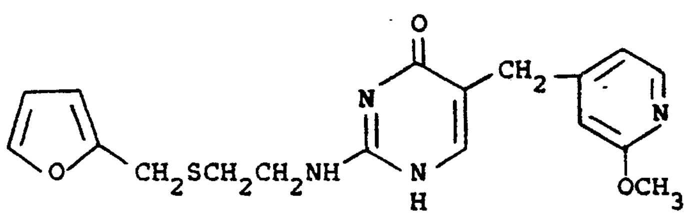

(VII)

mit einer organischen Lösung von bis(Dimethylamino)methan bei einem pH-Wert von 1,3 bis 2,5 umsetzt.

2. Verfahren nach Anspruch 1, bei dem der pH-Bereich 1,7 bis 2,3 ist.

3. Verfahren nach einem der Ansprüche 1 oder 2, das in Dichlormethan durchgeführt wird.

4. Verfahren nach Anspruch 1, bei dem der erforderliche pH-Wert durch Zugabe von Chlorwasserstoffgas erreicht wird.

5. Verfahren nach Anspruch 1, bei dem die Schutzgruppe der Verbindung der Formel VI abgespaltet wird, um 2-(2-(5-Dimethylaminomethylfuran-2-ylmethylthio)äthylamino)-5-(2-hydroxypyrid-4-ylmethyl)-pyrimidin-4-on oder ein pharmazeutisch verträgliches Salz davon zu erhalten.

**Revendications**

1. Un procédé pour la préparation d'un composé de la formule (VI):

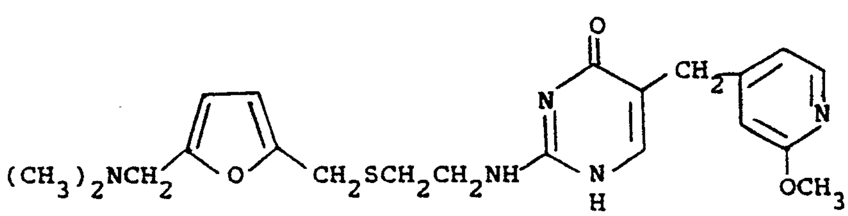

(VI)

qui comprend la mise en réaction d'un composé de la formule (VII):

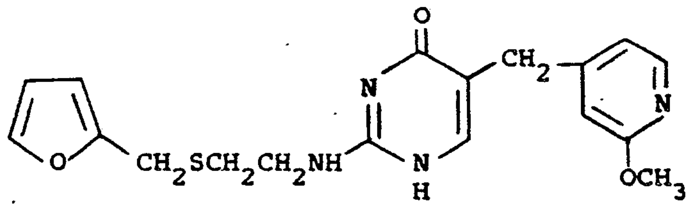

(VII)

avec une solution organique de bis(diméthylamino)méthane à un pH de 1,3 à 2,5.

2. Un procédé selon la revendication 1 dans lequel la gamme du pH est 1,7 à 2,3.

3. Un procédé selon la revendication 1 ou la revendication 2 lorsqu'il est réalisé dans du dichlorométhane.

4. Un procédé selon la revendication 1 dans lequel on obtient le pH exigé par l'ajout d'acide chlorhydrique gazeux.

5. Un procédé selon la revendication 1 dans lequel le composé de la formule (VI) est déprotégé afin de former 2-(2-(5-diméthylaminométhylfuranne-2-ylméthylthio)-éthylamino)-5-(2-hydroxypyrid-4-ylméthyle)-pyrimidine-4-un ou un sel du même pharmaceutiquement acceptable.